# EUROPEAN PATENT SPECIFICATION

(11) **EP 1 578 698 B1**
(45) Date of publication and mention of the grant of the patent: **07.03.2007**
(21) Application number: 02808240.2
(22) Date of filing: 16.12.2002
(51) Int. Cl.: C02F 5/00

(54) **STABILIZED AQUEOUS FOLIC ACID PREPARATION AND PROCESS FOR PROVIDING THE SAME**
STABILISIERTE WÄSSRIGE FOLSÄUREZUBEREITUNG UND VERFAHREN ZU DESSEN HERSTELLUNG
PREPARATION AQUEUSE D'ACIDE FOLIQUE STABILISEE ET SON PROCEDE DE PRODUCTION

(43) Date of publication of application: 28.09.2005
(73) Proprietor: Dosfolat AG, 9101 Herisau (CH)
(72) Inventor: Blum, Holger, 22301 Hamburg (DE)
(74) Representative: Kirschner, Klaus Dieter
(86) International application number: PCT/EP2002/014340
(87) International publication number: WO 2004/054936

(56) References cited:
- US-A- 5 624 564
- US-B1- 6 444 700

## Description

This invention relates to stabilized aqueous folic acid preparations and more specifically to aqueous vitamine preparations and a process for providing a stabilized aqueous dosage solution.

Combinations of folic acid or at least one ammonium, alkali metal, alkaline earth metal and/or alkanolammonium salt thereof and a water-soluble molybdenum compound in the form of dilute aqueous dosage solutions are known to be excellent additives for biological sewage treatment plants , as disclosed in US-A-5,624,564. Unfortunately, however, such diluted aqueous dosage solutions have been found to loose 30 to 50% of folic acid bioactivity within a few weeks, at best, or a few days at worst.

Up to this present time, the only practical method to overcome the problem of rapid loss of bioactivity of dilute dosage solutions containing folic acid or at least one ammonium, alkali metal, alkaline earth metal and/or alkanolammonium salt thereof in combination with a water-soluble molybdenum compound, known to the present inventor, have been to use dihydrofolic acid plus hydroxypolycarboxylic acid salts as stabilizers, as disclosed in US-A-4,931,442 or in US-A-4,956,092. Since these partially stabilized dosage solutions may have drawbacks such as increased cost due to the use of large amounts of dihydrofolic acid , an economical solution to the stability problem would be highly desired by biological sewage plant operators in order to maximize flexibility in using this combination of folic acid or at least one ammonium, alkali metal, alkaline earth metal and/or alkanolammonium salt thereof and a water-soluble molybdenum compound, in view of its excellent biostimulating activity.

This invention provides a stabilized aqueous folic acid preparation which comprises a combination of folic acid and/or dihydrofolic acid or at least one ammonium, alkali metal, alkaline earth metal and/or alkanolammonium salt and mixtures thereof and a water-soluble molybdenum compound, and which has an improved stability of its folic acid contents in the presence of oxygen, the said preparation containing as a stabilizer one polydental polyoxyethylene compound. The polydental polyoxyethylene compound is represented by the following formula (I) 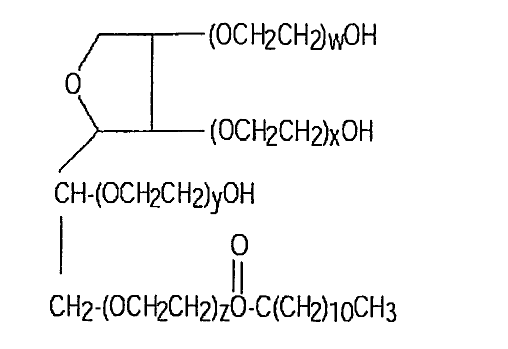 wherein w + x + y + z has a value of 15 to 25.

The applicant has now unexpectedly found that by using one specific polydental polyoxyethylene compound, the loss of vitamin bioactivity in dilute aqueous solutions comprising folic acid and/or dihydrofolic acid or at least one ammonium, alkali metal, alkaline earth metal and/or alkanolammonium salt thereof in combination with a water-soluble molybdenum compound is completely suppressed for several weeks time, resulting in easy to use dosage solutions for biological sewage treatment plants. This is really unexpected since the prior art does not teach or provide any method to counteract the loss of vitamin bioactivity of dilute aqeous dosage solutions containing folic acid or at least one ammonium, alkali metal, alkaline earth metal and/or alkanolammonium salt thereof in combination with a watersoluble molybdenum compound. Thus the stabilized folate dosing solution of the present invention consists of the following components and ingredients:
1) one member of the group selected from folic acid and/or dihydrofolic acid or at least one ammonium, alkali metal, alkaline earth metal and/or alkanolammonium salt thereof and
2) a water-soluble molybdenum compound,
3) a polydental polyoxyethylene compound which is represented by the formula (I) above wherein w + x + y + z has a value of 15 to 25. A compound wherein w + x + y + z has an average value of of 20 has CAS#9005-64-5 and is known as polyoxyethylene(20) sorbitan monolaurate or Polysorbate 20 NF or as TWEEN®20,
4) the folate dosing solution of the present invention optionally contains additives which have additional favorable influence on the stability in the form of hydroxypolycarboxylic acid and ammonium, alkali metal, alkaline earth metal and alkanolammonium salts thereof as disclosed in US-A-4,931,442.

According to a preferred embodiment of the invention the dilute aqueous folate dosage solutions contain the stabilizer of the formula(I) above preferably in an amount of approximate 0.1 to 1.2 mol per mol of folic acid and/or folic acid salts to be stabilized.

Dilute aqueous folate composition of the present invention are useful as additives to stimulate the creation of a healthy biomass and to create a compact sludge with good settability properties, for example in paper-, metals-, textiles- and plastics- and communal- biological sewage treatment plants.

The invention now will be explained in more detail by the following examples without, however, being restricted thereto. In the following Examples and Comparative Examples and in the Tables, the term "mMol" means "milli-Mol" that is 0.001 gram-Mol of the specified chemical compound. In the following Examples and Comparative Examples, the term "parts" means "parts by weight".

### REFERENCE EXAMPLE 1

### Preparation of Dosage Solution A

Into a 4-necked flask fitted with a stirrer and a submerged gas-dispersion tube were placed 14000 ml of distilled water and nitrogen was bubbled at ambient temperature through the water under constant stirring for one hour to remove all oxygen. Thereafter a preparation containing the sodium salt of folic acid, the sodium salt of dihydrofolic acid sodium molybdate dihydrate and sodium L-tartrate dihydrate was added to the water under nitrogen and stirring to produce a dosage solution. Dosage Solution A comprises the following compounds per 1000 g: 1.8 mMol of folic acid sodium salt , 0.45 mMol of dihydrofolic acid sodium salt, 0.2 mMol of sodium molybdate dihydrate, 0.9 mMol of sodium L-tartrate dihydrate.

The Dosage Solution A thus obtained was stored under a nitrogen blanket in brown glass bottles.

### REFERENCE EXAMPLE 2

### Preparation of Dosage Solution B

Into a 4-necked flask fitted with a stirrer and a submerged gas-dispersion tube were placed 12000 ml of distilled water and nitrogen was bubbled at ambient temperature through the water under constant stirring for one hour to remove all oxygen. Thereafter a preparation containing the TRIS salt of folic acid, the TRIS salt of dihydrofolic acid, sodium molybdate dihydrate and sodium L-tartrate dihydrate was added to the water under nitrogen and stirring to produce a dosage solution. Dosage Solution B comprises the following compounds per 1000 g: (TRIS is tris(hydroxymethyl)aminomethane, CAS#77-86-1) 1.82 mMol of folic acid TRIS salt, 1.65 mMol of dihydrofolic acid TRIS salt, 0.7 mMol of sodium molybdate dihydrate, 1.41 mMol of sodium L-tartrate dihydrate.

The Dosage Solution B thus obtained was stored under a nitrogen blanket in brown glass bottles.

### REFERENCE EXAMPLE 3

### Preparation of Dosage Solution C

Into a 4-necked flask fitted with a stirrer and a submerged gas-dispersion tube were placed 10000 ml of distilled water and nitrogen was bubbled at ambient temperature through the water under constant stirring for one hour to remove all oxygen. Thereafter a preparation containing the sodium salt of folic acid, the sodium salt of dihydrofolic acid, potassium molybdatocysteine salt and sodium L-tartrate dihydrate was added to the water under nitrogen and stirring to produce a dosage solution. Dosage Solution C comprises the following compounds per 1000 g: 1.73 mMol of folic acid sodium salt, 0.25 mMol of dihydrofolic acid sodium salt, 0.23 mMol of potassium molybdatocysteine salt "[MoO.sub.3 (Cys)]", 0.9 mMol of sodium L-tartrate dihydrate.

The Dosage Solution C thus obtained was stored under a nitrogen blanket in brown glass bottles.

### EXAMPLE 1

Into a 4-necked flask fitted with a stirrer and a submerged gas-dispersion tube, were placed 1000 parts of Dosage Solution A. To the solution was added under nitrogen and with stirring 0.73 parts of polyoxyethylene(20) sorbitan monolaurate, CAS#9005-64-5 to obtain Dosage Solution A-1, whose molar ratio of folic acid/polyoxyethylene(20) sorbitan monolaurate was 3 .

To simulate the conditions in a industrial dosage tank 100 ml of Dosage Solution A-1 was filled into a 250 ml narrow necked brown glass bottle closed with a screw cap so that 150 ml of air was present while the solution was stored for 20 days at ambient temperature.

### Evaluation of the folic acid vitamin stability :

Using the assay methods referred to in U.S. Patent No. 4,931,442 the stability of Dosage Solution A-1 was evaluated by drawing samples at the beginning and at the end of the 20 days storage period and microbiologically determining their folic acid contents and the stability was found to be 98%.

When 100 ml of Dosage Solution A was stored under 150 ml of air in a brown glass bottle for 20 days without the addition of polyoxyethylene(20) sorbitan monolaurate the stability was found to be only 62%.

### EXAMPLE 2

Into a 4-necked flask fitted with a stirrer and a submerged gas-dispersion tube were placed 1000 ml of distilled water. Thereafter a preparation which previously had been stored under nitrogen for 3 months time, containing the sodium salt of folic acid, the sodium salt of dihydrofolic acid, sodium molybdate dihydrate and sodium L-tartrate dihydrate and polyoxyethylene(20) sorbitan monolaurate was added to the water under stirring to produce Dosage Solution A-2. Dosage Solution A-2 comprises the following compounds per 1000 g: 1.8 mMol of folic acid sodium salt, 0.45 mMol of dihydrofolic acid sodium salt, 0.2 mMol of sodium molybdate dihydrate, 0.9 mMol of sodium L-tartrate dihydrate 0,59 mMol of polyoxyethylene(20) sorbitan monolaurate.

Dosage Solution A-2 and Dosage Solution A.-1 thus have identical composition. The only difference being that polyoxyethylene(20) sorbitan monolaurate was an ingredient in the original folic acid preparation used to make Dosage Solution A-2.

To simulate the conditions in a industrial dosage tank 100 ml of Dosage Solution A-2 was filled into a 250 ml narrow necked brown glass bottle closed with a screw cap so that 150 ml of air was present while the solution was stored for 20 days at ambient temperature.

### Evaluation of the folic acid vitamin stability :

Using the assay methods referred to in U.S. Patent No. 4,931,442 the stability of Dosage Solution A-2 was evaluated by drawing samples at the beginning and at the end of the 20 days storage period and microbiologically determining their folic acid contents and the stability was found to be 98% and therefore identical to that found with Dosage Solution A-1.

### EXAMPLES 3 to 18

Productive examples of dilute aqueous folate dosage solutions compositions were formed similarly to Example 1 in accordance with the formulations specified in Table 1 (TWEEN®20 was used as polyoxyethylene(20) sorbitan monolaurate)

| **TABLE 1** E**xample #** | Dosage Solution | mMol folic acid in 1000gramm of dosage sol. at start of test | Ethoxylate# | Mol-ethoxylate per Mol folic acid added to dosage solution | %Biostability after 20 days storage |
|---|---|---|---|---|---|
| 3 | A | 1,8 | #1 | 0,15 | 87 |
| 4 | A | 1,8 | #1 | 0,6 | 98 |
| 5 | A | 1,8 | #1 | 1,2 | 98 |
| 6 | A | 1,8 | #2 | 0,33 | 94 |
| 7 | A | 1,8 | #2 | 0,7 | 94 |
| 8 | A | 1,8 | #3 | 0,34 | 96 |
| 9 | A | 1,8 | #3 | 0,68 | 90 |
| 10 | B | 1,82 | | | 73 |
| 11 | B | 1,82 | #1 | 0,2 | 87 |
| 12 | B | 1,82 | #2 | 0,2 | 86 |
| 13 | B | 1,82 | #3 | 0,2 | 82 |
| 14 | C | 1,73 | | | 67 |
| 15 | C | 1,73 | #1 | 0,33 | 99 |
| 16 | C | 1,73 | #1 | 0,7 | 99 |
| 17 | C | 1,73 | #2 | 0,4 | 89 |
| 18 | C | 1,73 | #3 | 0,4 | 84 |
| | | | | | |

In Table 1, Ethoxylate1 is polyoxyethylene(20) sorbitan monolaurate CAS#9005-64-5 Ethoxylate 2 is polyoxyethylene (15) sorbitan monolaurate, a compound which is represented by the formula (I) above wherein w + x + y + z has an average value of 15.

Ethoxylate 3 is polyoxyethylene (25) sorbitan monolaurate, a compound which is represented by the formula (I) above wherein w + x + y + z has an average value of 25.

### COMPARATIVE EXAMPLE D1

Into a 4-necked flask fitted with a stirrer and a submerged gas-dispersion tube, were placed 1000 parts of Dosage Solution A .To the solution was added under nitrogen and with stirring 0.78 parts of polyoxyethylene(20) sorbitan monooleate, CAS#9005-65-6 which contains about 20 moles of ethylenoxide per mol , to obtain Dosage Solution D-1, whose molar ratio of folic acid/polyoxyethylene compound was 3 .

To simulate the conditions in a industrial dosage tank 100 ml of Dosage Solution D-1 was filled into a 250 ml narrow necked brown glass bottle closed with a screw cap so that 150 ml of air was present while the solution was stored for 20 days at ambient temperature.

### Evaluation of the folic acid vitamin stability :

Using the assay methods referred to in U.S. Patent No. 4,931,442 the stability of Dosage Solution D-1 was evaluated by drawing samples at the beginning and at the end of the 20 days storage period and microbiologically determining their folic acid contents and the stability was found to be 78%.

### COMPARATIVE EXAMPLES D2 to D17

Dosage Solutions D-2 to D-17 were formed similarly to the procedure of Comparative Example 1 in accordance with the formulations specified in Table 2 (the amount of each polyethoxylate component is expressed by "moles ethoxylate per mol of folic acid ").

| **TABLE 2 Comparative Example#** | Dosage Solution | mMol folic acid in 1000gramm of dosage sol. at start of test | Ethoxylat# | Mol-ethoxylate per Mol folic acid added to dosage solution | %Biostability after 20 days storage |
|---|---|---|---|---|---|
| 2 | A | 1,8 | #4 | 0,6 | 77 |
| 3 | B | 1,82 | #4 | 0,6 | 74 |
| 4 | A | 1,8 | #5 | 0,6 | 72 |
| 5 | B | 1,82 | #5 | 0,6 | 79 |
| 6 | C | 1,73 | #5 | 0,6 | 74 |
| 7 | A | 1,8 | #6 | 0,6 | 76 |
| 8 | B | 1,82 | #7 | 0,6 | 56 |
| 9 | B | 1,82 | #7 | 0,6 | 60 |
| 10 | B | 1,82 | #8 | 0,6 | 48 |
| 11 | C | 1,73 | #9 | 0,6 | 55 |
| 12 | B | 1,82 | #9 | 0,6 | 59 |
| 13 | A | 1,8 | #10 | 0,6 | 79 |
| 14 | B | 1,82 | #10 | 0,6 | 79 |
| 15 | C | 1,73 | #10 | 0,6 | 75 |
| 16 | A | 1,8 | #11 | 0,6 | 72 |
| 17 | C | 1,73 | #11 | 0,6 | 70 |
| | | | | | |

In Table 2, Ethoxylate4 is polyoxyethylene(20) sorbitan monooleate, CAS#9005-65-6, Ethoxylate5 is polyoxyethylene(20) sorbitan monopalmitate,CAS#9005-66-7, Ethoxylate6 is polyoxyethylene(20) sorbitan monostearate, CAS#9005-67-8, Ethoxylate7 is polyoxyethylene(20) sorbitan trioleate, CAS#9005-70-3, Ethoxylate8 is polyoxyethylene(10) isooctylphenyl ether, CAS#9002-93-1, and Ethoxylate9 is polyoxyethylene(40) isooctylphenyl ether, 70% solution in water. Ethoxylate10 is polyoxyethylene (12.7) sorbitan monolaurate, a compound which is represented by the formula (I) above wherein w + x + y + z has an average value of 12.7, and Ethoxylate 11 is polyoxyethylene (33) sorbitan monolaurate, a compound which is represented by the formula (I) above wherein w + x + y + z has an average value of 33.

While the invention has been described above with references to specific embodiments thereof, it is apparent that many changes, modifications and variations in the material, arrangements of parts and steps can be made without departing from the inventive concept disclosed herein. Accordingly, the scope of the appended claims is intended to embrace all such changes, modifications and variations that may occur to one of skill in the art upon a reading of the disclosure.

## Claims

1. An aqueous folic acid dosage solution stabilized against loss of bioactivity comprising:
a) folic acid and/or dihydrofolic acid or at least one ammonium, alkali metal, alkaline earth metal and/or alkanolammonium salt thereof, and mixtures thereof; and
b) a water-soluble molybdenum compound, which has improved stability of its folic acid contents in the presense of oxygen, the said dosage solution containing as a stabilizer one polydental polyoxyethylene compound of the formula (I) 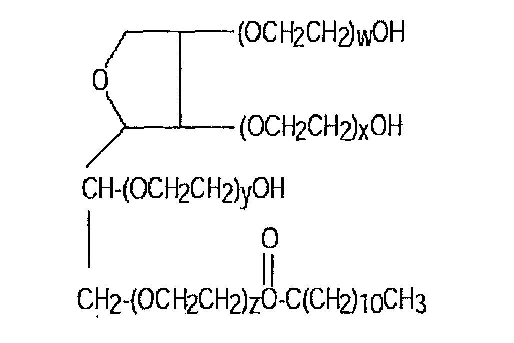
wherein w + x + y + z has a value of 15 to 25.

2. The dosage solution of claim 1, wherein said polydental polyoxyethylene compound is present in an amount between approximate 0.1 gramm-Mol and 1.2 gramm-Mol per gramm-Mol of folic acid of said preparation.

3. The dosage solution of claim 1, wherein said polydental polyoxyethylene compound is polyoxyethylene(20) sorbitan monolaurate.

4. A process for providing a stabilized aqueous folic acid dosage solution containing folic acid and/or dihydrofolic acid or at least one ammonium, alkali metal, alkaline earth metal and/or alkanolammonium salt thereof, and mixtures thereof and a water-soluble molybdenum compound which comprises the steps of:
a) mixing first said polydental polyoxyethylene compound of the formula (I) 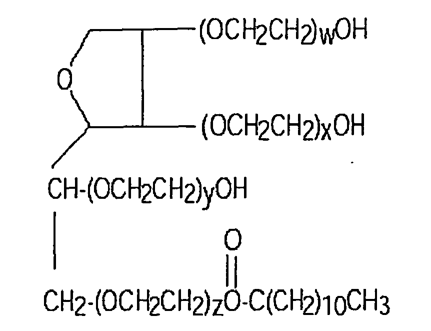 wherein w + x + y + z has a value of 15 to 25 with the dilution water; and thereafter
b) adding to the same dilution water a preparation comprising; p1) folic acid and/or dihydrofolic acid or at least one ammonium, alkali metal, alkaline earth metal and/or alkanolammonium salt thereof, and mixtures thereof and p2) a water-soluble molybdenum compound and optionally; p3) one member selected from the group consisting of at least one hydroxypolycarboxylic acid and ammonium, alkali metal, alkaline earth metal and alkanolammonium salts thereof.

5. A process for providing a stabilized aqueous folic acid dosage solution containing folic acid and/or dihydrofolic acid or at least one ammonium, alkali metal, alkaline earth metal and/or alkanolammonium salt thereof ,and mixtures thereof and a water-soluble molybdenum compound which comprises the steps of:
a) preparing a pre-mix of p1) folic acid and/or dihydrofolic acid or at least one ammonium, alkali metal, alkaline earth metal and/or alkanolammonium salt thereof and of p2) a water-soluble molybdenum compound, and of p3) one polydental polyoxyethylene compound of the formula (I) 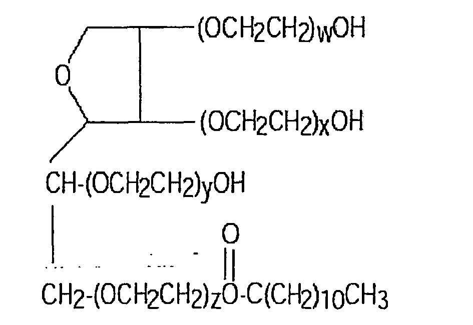 wherein w + x + y + z has a value of 15 to 25, and optionally of p4) one member selected from the group consisting of at least one hydroxypolycarboxylic acid and ammonium, alkali metal, alkaline earth metal and alkanolammonium salts thereof, and
b) mixing batchwise one part of said pre-mix; 5a) with approximate one to 400 parts of dilution water.

6. A process for providing a stabilized aqueous folic acid dosage solution containing;
a) folic acid and/or dihydrofolic acid or at least one ammonium, alkali metal, alkaline earth metal and/or alkanolammonium salt thereof ,and mixtures thereof and b) a water-soluble molybdenum compound which comprises the steps of:
a) preparing a pre-mix of; p1) folic acid and/or dihydrofolic acid or at least one ammonium, alkali metal, alkaline earth metal and/or alkanolammonium salt thereof and of p2) a water-soluble molybdenum compound, and of p3) one polydental polyoxyethylene compound of the formula (I) 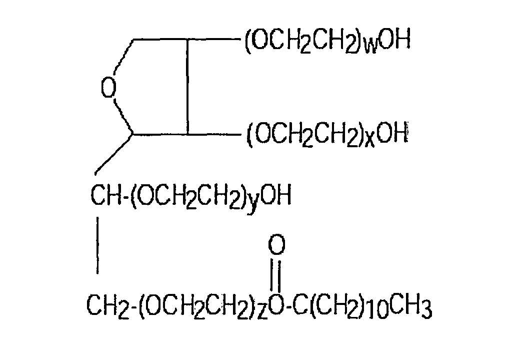 wherein w + x + y + z has a value of 15 to 25 and optionally of p4) one member selected from the group consisting of at least one hydroxypolycarboxylic acid and ammonium, alkali metal, alkaline earth metal and alkanolammonium salts thereof, and
b) mixing continously one part of said pre-mix 6a) with approximate one to 400 parts of dilution water.

## Patentansprüche

1. Wässrige Folsäuredosierlösung, die gegen den Verlust von Bioaktivität stabilisiert ist, enthaltend:
a) Folsäure und/oder Dihydrofolsäure oder mindestens ein Ammonium-, Alkalimetall-, Erdalkalimetall- und/oder Alkanolammoniumsalz davon, und Gemische daraus, und
b) eine wasserlösliche Molybdänverbindung,
die eine verbesserte Stabilität ihres Folsäuregehaltes in Gegenwart von Sauerstoff aufweist,
wobei die Dosierlösung als Stabilisator eine mehrzähnige Polyoxyethylenverbindung der Formel (I) enthält: 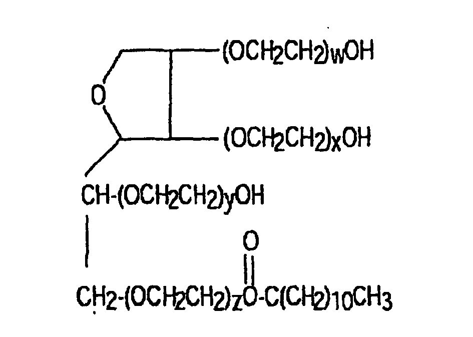 in der w+x+y+z einen Wert von 15 bis 25 hat.

2. Dosierlösung nach Anspruch 1, wobei die mehrzähnige Polyoxyethylenverbindung in einer Menge zwischen ungefähr 0,1 Gramm-Mol und 1,2 Gramm-Mol pro Gramm-Mol Folsäure der Präparation vorhanden ist.

3. Dosierlösung nach Anspruch 1, wobei die mehrzähnige Polyoxyethylenverbindung Polyoxyethylen(20)sorbitanmonolaurat ist.

4. Verfahren zur Bereitstellung einer stabilisierten wässrigen Folsäuredosierlösung, die Folsäure und/oder Dihydrofolsäure oder mindestens ein Ammonium-, Alkalimetall-, Erdalkalimetall- und/oder Alkanolammoniumsalz davon, und Gemische daraus, und eine wasserlösliche Molybdänverbindung enthält, bei dem:
a) zuerst die mehrzähnige Polyoxyethylenverbindung der Formel (I) 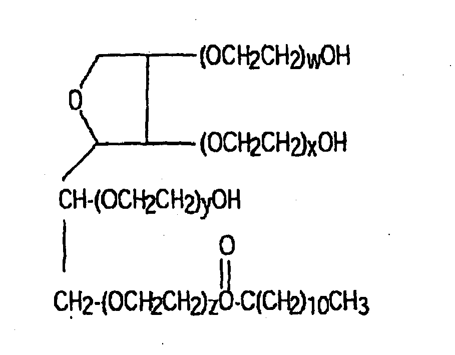 in der w+x+y+z einen Wert von 15 bis 20 hat, mit dem Verdünnungswasser vermischt wird und danach
b) zu dem gleichen Verdünnungswasser eine Präparation gegeben wird, die p1) Folsäure und/oder Dihydrofolsäure oder mindestens ein Ammonium-, Alkalimetall-, Erdalkalimetall und/oder Alkanolammoniumsalz davon, oder Gemische daraus, und p2) eine wasserlösliche Molybdänverbindung und ggf. p3) ein Mitglied der aus mindestens einer Hydroxypolycarbonsäure und Ammonium- Alkalimetall-, Erdalkalimetall- und Alkanolammoniumsalze davon enthaltenden Gruppe, enthält.

5. Verfahren zur Bereitstellung einer stabilisierten wässrigen Folsäuredosierlösung, die Folsäure und/oder Dihydrofolsäure oder mindestens ein Ammonium-, Alkalimetall-, Erdalkalimetall- und/oder Alkanolammoniumsalz davon, und Gemische daraus, und eine wasserlösliche Molybdänverbindung enthält, bei dem:
a) ein Vorgemisch aus p1) Folsäure und/oder Dihydrofolsäure oder mindestens einem Ammonium-, Alkalimetall-, Erdalkalimetall- und/oder Alkanolammoniumsalz davon und p2) einer wasserlöslichen Molybdänverbindung und p3) einer mehrzähnigen Polyoxyethylenverbindung der Formel (I) 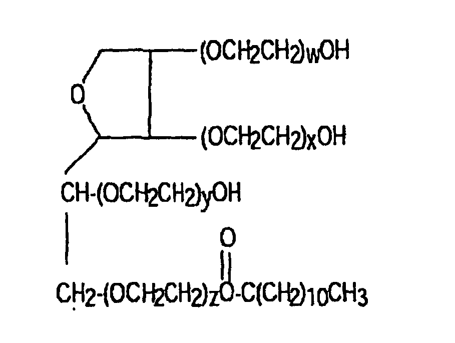 in der w+x+y+z ein Wert von 15 bis 25 hat, und ggf. p4) einem Mitglied der aus mindestens einer Hydroxypolycarbonsäure und Ammonium-, Alkalimetall-, Erdalkalimetall- und Alkanolammoniumsalzen davon bestehenden Gruppe hergestellt wird, und
b) chargenweise ein Teil des Vorgemisches 5a) mit ungefähr 1 bis 400 Teilen Verdünnungswasser vermischt wird.

6. Verfahren zur. Bereitstellung einer stabilisierten wässrigen Folsäuredosierlösung, die a) Folsäure und/oder Dihydrofolsäure oder mindestens ein Ammonium-, Alkalimetall-, Erdalkalimetall- und/oder Alkanolammoniumsalz davon, und Gemische daraus, und b) eine wasserlösliche Molybdänverbindung enthält, bei dem:
a) ein Vorgemisch aus p1) Folsäure und/oder Dihydrofolsäure oder mindestens einem Ammonium-, Alkalimetall-, Erdalkalimetall- und/oder Alkanolammoniumsalz davon und p2) einer wasserlöslichen Molybdänverbindung und p3) einer mehrzähnigen Polyoxyethylenverbindung der Formel (I) 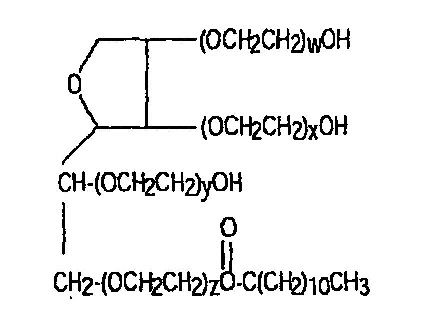 in der w+x+y+z einen Wert von 15 bis 25 hat, und ggf. p4) einem Mitglied aus der aus mindestens einer Hydroxypolyarbonsäure und Ammonium-, Alkalimetall-, Erdalkalimetall- und Alkanolammoniumsalzen davon bestehenden Gruppe hergestellt wird, und
b) kontinuierlich ein Teil des Vorgemisches 6a) mit ungefähr 1 bis 400 Teilen Verdünnungswasser vermischt wird.

## Revendications

1. Solution aqueuse de dosage d'acide folique stabilisée contre la perte de bioactivité comprenant:
a) l'acide folique et/ou l'acide dihydrofolique ou au moins un ammonium, un métal alcalin, un métal alcalino-terreux et/ou un sel d'alkanolammonium de ceux-ci, et des mélanges de ceux-ci; et
b) un composé de molybdène soluble dans l'eau, qui a amélioré la stabilité de ses teneurs d'acide folique dans la présence d'oxygène, ladite solution de dosage contenant comme un stabilisateur un composé de polyoxyéthylène polydental de la formule (I) 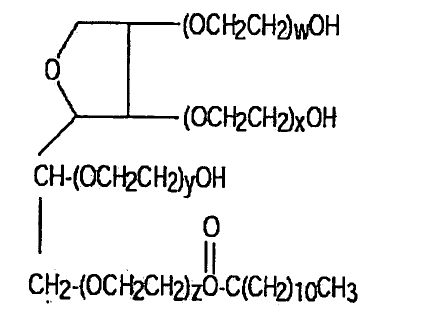
où w + x + y + z a une valeur de 15 à 25.

2. Solution de dosage selon la revendication 1, où ledit composé de polyoxyéthylène polydental est présent dans une quantité entre environ 0,1 gramme-mole et 1,2 gramme-mole par gramme-mole d'acide folique de ladite préparation.

3. Solution de dosage selon la revendication 1, où ledit composé de polyoxyéthylène polydental est le monolaurate de polyoxyéthylène (20) sorbitane.

4. Un procédé de production une solution aqueuse de dosage d'acide folique stabilisée contenant l'acide folique et/ou l'acide dihydrofolique ou au moins un ammonium, un métal alcalin, un métal alcalino-terreux et/ou un sel d'alkanolammonium de ceux-ci, et des mélanges de ceux-ci et un composé de molybdène soluble dans l'eau, qui comprend les étapes de:
a) mélanger d'abord ledit composé de polyoxyéthylène polydental de la formule (I) 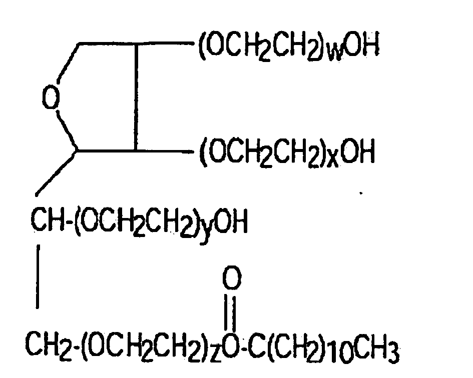 où w + x + y + z a une valeur de 15 à 25 avec l'eau de dilution; et après
b) ajouter à la même eau de dilution une préparation comprenant; p1) l'acide folique et/ou l'acide dihydrofolique ou au moins un ammonium, un métal alcalin, un métal alcalino-terreux et/ou un sel d'alkanolammonium de ceux-ci, et des mélanges de ceux-ci et p2) un composé de molybdène soluble dans l'eau et facultativement; p3) un élément choisi du groupe formé d'au moins un acide hydroxypolycarboxylique et d'ammonium, du métal alcalin, du métal alcalino-terreux et des sels d'alkanolammonium de ceux-ci.

5. Un procédé de production une solution aqueuse de dosage d'acide folique stabilisée contenant l'acide folique et/ou l'acide dihydrofolique ou au moins un ammonium, un métal alcalin, un métal alcalino-terreux et/ou un sel d'alkanolammonium de ceux-ci, et des mélanges de ceux-ci et un composé de molybdène soluble dans l'eau, qui comprend les étapes de:
a) préparer un pré-mélange de p1) l'acide folique et/ou l'acide dihydrofolique ou au moins un ammonium, un métal alcalin, un métal alcalino-terreux et/ou un sel d'alkanolammonium de ceux-ci, et de p2) un composé de molybdène soluble dans l'eau, et de p3) un composé de polyoxyéthylène polydental de la formule (I) 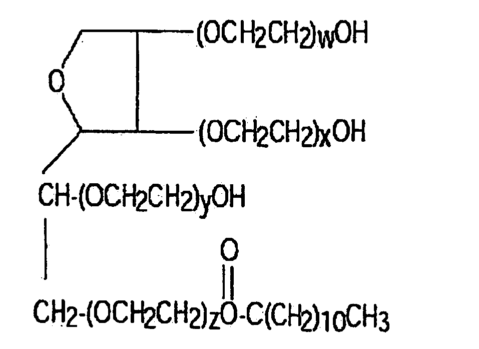 où w + x + y + z a une valeur de 15 à 25, et facultativement de p4) un élément choisi d'un groupe formé d'au moins un acide hydroxypolycarboxylique et d'ammonium, du métal alcalin, du métal alcalino-terreux et/ou des sels d'alkanolammonium de ceux-ci, et
b) mélanger de manière discontinue une part dudit pré-mélange; 5a) avec environ une à 400 parts d'eau de dilution.

6. Un procédé de production une solution aqueuse de dosage d'acide folique stabilisée contenant;a) l'acide folique et/ou l'acide dihydrofolique ou au moins un ammonium, un métal alcalin, un métal alcalino-terreux et/ou un sel d'alkanolammonium de ceux-ci, et des mélanges de ceux-ci et b) un composé de molybdène soluble dans l'eau, qui comprend les étapes de:
a) préparer un pré-mélange de; p1) l'acide folique et/ou l'acide dihydrofolique ou au moins un ammonium, un métal alcalin, un métal alcalino-terreux et/ou un sel d'alkanolammonium de ceux-ci, et de p2) un composé de molybdène soluble dans l'eau, et de p3) un composé de polyoxyéthylène polydental de la formule (I) 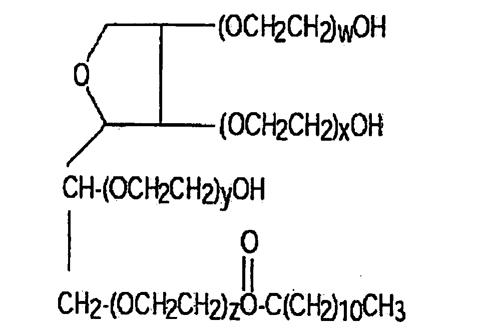 où w + x + y + z a une valeur de 15 à 25, et facultativement de p4) un élément choisi d'un groupe formé d'au moins un acide hydroxypolycarboxylique et d'ammonium, du métal alcalin, du métal alcalino-terreux et/ou des sels d'alkanolammonium de ceux-ci, et
b) mélanger de manière continue une part dudit pré-mélange 6a) avec environ une à 400 parts d'eau de dilution.
